Europäisches Patentamt

(19) European Patent Office    (11) Numéro de publication: **0 133 828**

Office européen des brevets    **B1**

(12)    # FASCICULE DE BREVET EUROPÉEN

(45) Date de publication du fascicule du brevet:    (51) Int. Cl. ⁵: **A 61 N    1/36**
28.02.90

(21) Numéro de dépôt: **84401536.2**

(22) Date de dépôt: **20.07.84**

(54) Procédé de commande d'un stimulateur cardiaque.

(30) Priorité: **02.08.83 FR 8312740**

(43) Date de publication de la demande:
**06.03.85 Bulletin 85/10**

(45) Mention de la délivrance du brevet:
**28.02.90 Bulletin 90/09**

(84) Etats contractants désignés:
**AT BE CH DE GB IT LI LU NL SE**

(56) Documents cité:
**EP-A-0 096 464**
**DE-A-2 609 365**
**FR-A-2 516 797**
**US-A-3 867 950**
**US-A-3 996 928**

(73) Titulaire: **BIOVALLEES Société Anonyme:**
**F-43150 Monastier sur Gazelle (FR)**

(72) Inventeur: **Brehler, Jacques**
**26, rue Barbier**
**F-72000 Le Mans (FR)**

(74) Mandataire: **Derambure, Christian**
**BUGNION ASSOCIES 55, rue Boissonade**
**F-75014 Paris (FR)**

EP 0 133 828 B1

## Description

La présente invention concerne les stimulateurs cardiaques implantables.

Les stimulateurs cardiaques implantables sont réglés ou programmés pour stimuler le coeur d'un patient selon une certaine fréquence fixée. Il existe certains stimulateurs cardiaques qui peuvent être reprogrammés par voie externe pour différentes fréquences de stimulation. Cependant, les stimulateurs cardiaques connus ne sont pas conçus pour être adaptés au degré d'activité physique réelle du sujet porteur chez qui le stimulateur est implanté. Cela signifie que si le patient exerce une activité physique importante, la fréquence de programmation préfixée peut se révéler insuffisante avec les conséquences de fatigue du patient qui en résultent. Au contraire, lorsque par exemple, le patient est au repos, ou endormi, la fréquence de stimulation préprogrammée peut être excessivement élevée, de façon superflue, voire dangereuse. Ainsi, la fréquence de stimulation choisie est un compromis qui n'autorise, à partir de la valeur choisie qu'une faible variation du degré d'activité physique du patient.

On connaît aussi (DE 2 609 365) un stimulateur cardiaque réglable, qui, cependant ne permet pas de résoudre le problème de la sécurité nécessaire lors de la détermination de la fréquence lorsque celle-ci croît du fait de l'augmentation de la température du patient.

L'invention a pour objectif de remédier à ces inconvénients. A cet effet, l'invention propose un procédé, et un stimulateur permettant au coeur du patient d'être stimulé à une fréquence correspondant à celle requise par le degré d'activité physique réel et actuel.

Selon l'invention, le coeur est stimulé à une fréquence de base correspondant à l'exigence du patient à un degré normal d'activité physique: on mesure la température du patient en tant que paramètre lié au degré d'activité physique du patient et on modifie la fréquence de stimulation de telle manière que la fréquence de stimulation est augmentée ou diminuée lorsque la température est respectivement augmentée ou diminuée, la fréquence de stimulation varie entre des limites supérieure et inférieure. La fréquence de stimulation est inférieure à la fréquence limite supérieure si la température excède une température limite.

Le paramètre mesuré est la température du patient, plus particulièrement celle du sang dans le coeur.

Egalement, selon une autre caractéristique, lorsque la température excède la borne supérieure, la fréquence de stimulation est diminuée par rapport à sa limite supérieure, par exemple de 20 %.

L'invention concerne également un stimulateur cardiaque implantable mettant en oeuvre le procédé qui vient d'être décrit. Un tel stimulateur cardiaque comporte des moyens de mesure de la température du patient, des moyens répondant au changement de la température pour augmenter ou diminuer la fréquence de stimulation, des moyens pour établir des limites supérieure et inférieure à la dite fréquence de stimulation et des moyens pour imposer une fréquence inférieure à la fréquence limite supérieure si la température excède une température limite.

L'invention sera bien comprise grâce à la description qui suivra, en référence aux dessins annexés dans lesquels :

- la figure 1 est un bloc diagramme de la partie électronique d'un stimulateur cardiaque implantable, selon l'invention,
- la figure 2 est une vue en coupe longitudinale illustrant la partie extrême de stimulation d'une sonde associée à un stimulateur cardiaque,
- la figure 3 est un diagramme illustrant la relation entre la température mesurée et la fréquence de stimulation dans un stimulateur cardiaque selon l'invention.

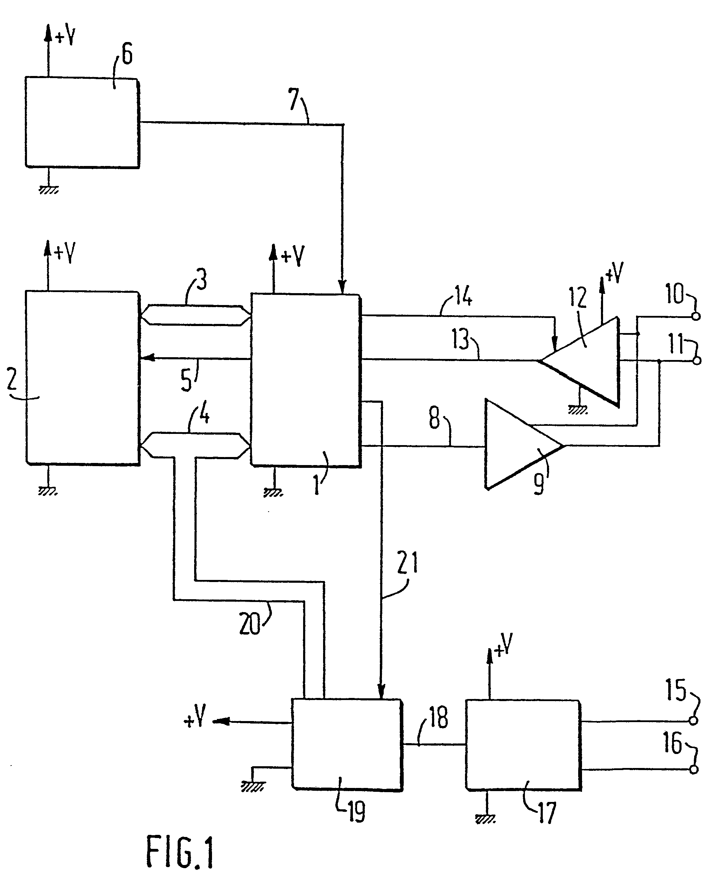

Un stimulateur cardiaque, selon l'invention comprend, outre les éléments conventionnels connus, le circuit électronique représenté sur la figure 1 comportant un microprocesseur 1 connecté à une mémoire de lecture 2 au moyen d'un bus d'adresses 3, un bus de données 4 et une ligne de commande 5. Une horloge 6 génératrice d'impulsions et reliée par une ligne 7 à une entrée du microprocesseur 1. Le microprocesseur 1 a une ligne de sortie 8 reliée à l'entrée d'un amplificateur de stimulation 9 dont les sorties sont reliées aux bornes 10 et 11 auxquelles est branchée une sonde de stimulation (sa partie électrique de stimulation).

Les bornes 10 et 11 sont également reliées aux entrées d'un d'un amplificateur de détection 12 dont la sortie est reliée par une ligne 13 au microprocesseur 1. Le microprocesseur 1 a également une sortie reliée par une ligne 14 à une entrée de commande de l'amplificateur de détection 12.

Deux entrées 15 et 16 sont prévues pour être reliées à une thermistance décrite ultérieurement. Les entrées 15 et 16 sont également reliées aux entrées d'un convertisseur analogique-digital 17. La sortie du convertisseur 17 est reliée par une ligne 18, à l'entrée d'un compteur 19. Les sorties du compteur 19 sont reliées par un bus 20 au bus de données 4. Enfin, le compteur 19 est commandé par le microprocesseur 1 par une ligne 21.

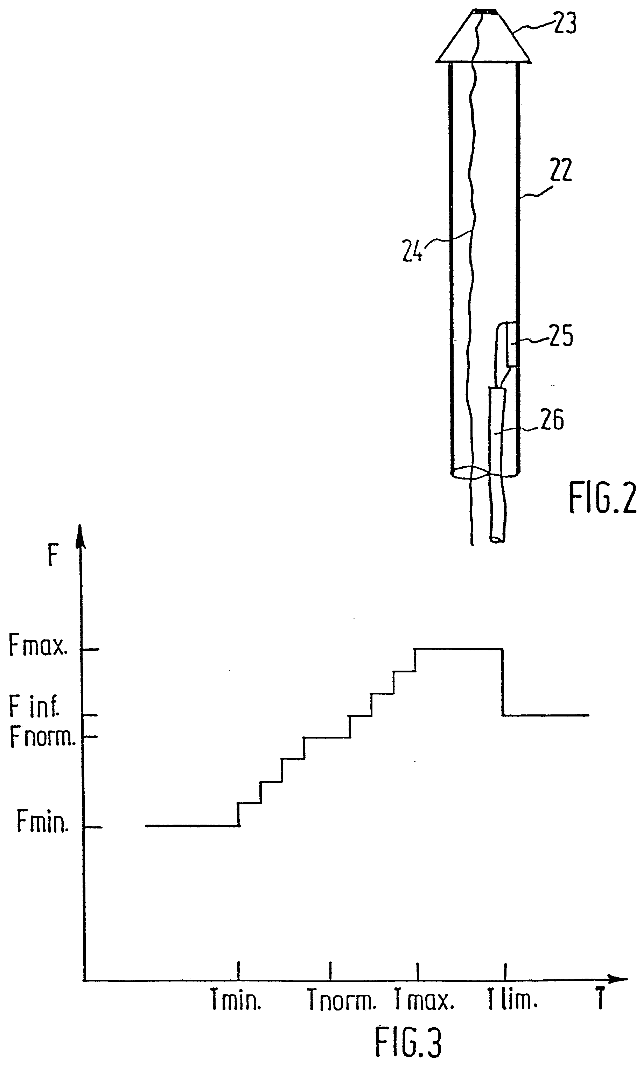

Sur la figure 2 est représentée la partie extrême libre de stimulation d'une sonde 22 comportant une extrémité de stimulation 23. Celle-ci est reliée à l'une des bornes 10, 11, par l'intermédiaire d'un fil conducteur spiralé 24, l'ensemble étant placé dans une gaine. L'autre borne 10, 11 peut être reliée à une autre électrode, par exemple le boîtier de stimulateur cardiaque formant masse. A la surface externe de la gaine est placée une thermistance 25 faisant partie intégrante de la sonde 22. Cette thermistance 25 est placée, de telle manière qu'elle permet une mesure effective du corps qui l'environne, par exemple le sang du coeur du patient. A cette thermistance 25 sont branchés des conducteurs 26 également noyés dans la gaine, connectés aux bornes 15 et 16 (figure 1).

Préférentiellement, la thermistance 25 est écartée de l'extrémité de stimulation 23, par exemple d'une distance de l'ordre de 10 cm, ce qui permet une mesure correcte de la température, la thermistance étant placée dans l'oreillette droite à proximité immédiate de l'abouchement de la veine cave inférieure. La résistance de la thermistance 25 varie en fonction de la température du média qui l'environne. Cela provoque une variation du courant d'entrée du convertisseur 17 qui cause une variation correspondante de la fréquence du signal de sortie du convertisseur 17. Le microprocesseur 1 échantillonne la température en activant le compteur 19, via la ligne de commande 21 pendant une période prédéterminée. La lecture en sortie du compteur 19 est transférée au microprocesseur 1 via le bus 20 et le bus 4. Le microprocesseur 1 ajuste la fréquence de sortie pour l'amplificateur de stimulation 9 via la ligne 8, selon la valeur lue au compteur 19 et les fréquences de stimulation-préprogrammées se trouvant dans la mémoire 2.

On se réfère maintenant à la figure 3 qui est un diagramme donnant en abscisses la température du sang T

- correspondant au degré d'activité physique du patient,
- mesurée par la thermistance 25,

et en ordonnées, la fréquence de stimulation F. A une température Tnorm qui est une température considérée normale pour le patient, correspond une fréquence de stimulation Fnorm. Si la température du patient augmente à partir de Tnorm, du fait de l'activité physique notamment, la fréquence de stimulation va également augmenter et ce jusqu'à une fréquence maximale Fmax correspondant à une température Tmax. De la même manière, si la température du sang diminue, à partir de la température Tnorm, la fréquence de stimulation diminue et ce jusqu'à une fréquence de stimulation minimale Fmin correspondant à une température Tmin. Si la température continue à diminuer à partir de Tmin, la fréquence restera inchangée à la valeur Fmin. D'autre part, si la température augmente à partir de la valeur Tmax, la fréquence restera inchangée à la valeur Fmax jusqu'à ce que la température atteigne une limite Tlim. A partir et au dessus de Tlim, la fréquence de stimulation est ramenée à une valeur inférieure à Fmax, soit Finf qui est, par exemple, de l'ordre de 20 % inférieure à la fréquence Fmax. Tlim peut être par exemple choisie entre 38°C et 39°C, préférentiellement égale ou voisine de 38,5°C (Tnorm étant égal ou de l'ordre de 37°C). La fréquence Finf peut être, par exemple inférieure de 15 à 25 % à la fréquence Fmax.

Il est clair que les températures Tlim, Tnorm, Tmax ainsi que les fréquences Flim, Fnorm, Fmax et Finf peuvent être préprogrammées ou réglées également par voie externe ou interne.

On peut également concevoir que la fonction liant la fréquence à la température puisse être programmée. Dans la variante illustrée sur la figure, cette fonction est illustrée, dans sa partie centrale, par un escalier, c'est-à-dire que la fréquence reste constante dans la plage de température et fait un saut lorsque la température atteint la borne de la plage. Mais, on peut concevoir également un réglage continu de la température.

Pour éviter la prise en compte de brusques variations de température, il peut être prévu des rayons aptes à effectuer une moyenne de température ou encore à lisser la courbe de variation de température.

## Revendications

1. Procédé de commande d'un stimulateur cardiaque implantable, dans lequel:

on stimule le coeur à une fréquence de base correspondant à l'exigence du patient à un degré normal d'activité physique;

on mesure la température du patient en tant que paramètre lié au degré d'activité physique du patient;

on modifie la fréquence de stimulation de telle manière que la fréquence de stimulation est augmentée ou diminuée quand la température est respectivement augmentée ou diminuée, la fréquence de stimulation varie entre des limites supérieure et inférieure,

caractérisé par le fait que la fréquence de stimulation est inférieure à la fréquence limite supérieure si la température excède une température limite.

2. Procédé selon la revendication 1, caractérisé en ce que la température mesurée est celle du sang du patient.

3. Procédé selon la revendication 2, caractérisé en ce que la température est mesurée dans le coeur du patient.

4. Procédé selon l'une quelconque des revendications 1 à 3, caractérisé en ce que la température limite est comprise entre 38 et 39°C, préférentiellement égale ou voisine de 38,5°C.

5. Procédé selon l'une quelconque des revendications 1 à 4, caractérisé en ce que la fréquence inférieure est inférieure de 15 à 25 %, préférentiellement de 20 % à la fréquence limite supérieure.

6. Stimulateur cardiaque implantable, comprenant des moyens (25, 17, 19) pour mesurer la température du patient, des moyens (1, 2) répondant au changement de la température pour augmenter ou diminuer la fréquence de stimulation, des moyens pour établir des limites supérieure et inférieure à ladite fréquence de stimulation caractérisé en ce qu'il comporte des moyens pour imposer une fréquence inférieure à la fréquence limite supérieure si la température excède une température limite.

7. Stimulateur cardiaque selon la revendication 6, caractérisé par le fait que les moyens de mesure comportent une thermistance (25) reliée à un circuit électronique (17, 19), adaptée pour mesurer la température du sang du patient.

8. Stimulateur cardiaque selon la revendication 7, caractérisé par le fait que la thermistance (25) est portée par la sonde de stimulation (22) reliée au stimulateur cardiaque.

9. Stimulateur cardiaque selon l'une quelconque des revendications 6 à 8, caractérisé par le fait que le circuit électronique relié à la thermistance (25) comprend un convertisseur analogique-digital (17) relié à un compteur (19), qui est relié à un microprocesseur (1) par un bus (20), le microprocesseur (1) étant relié au compteur (19) par une ligne de commande (21).

**Patentansprüche**

1. Verfahren zum Steuern eines implantierbaren Herzschrittmachers, bei welchem:

das Herz mit einer Grundfrequenz, die der Anforderung eines Patienten mit normaler physischer Aktivität entspricht, stimuliert wird,

bei welchem die Temperatur des Patienten als mit dem Grad der physischen Aktivität des Patienten verbundener Parameter gemessen wird und

bei welchem die Stimulationsfrequenz derart modifiziert wird, daß sie erhöht oder verringert wird, wenn sich die Temperatur erhöht oder verringert, wobei sich die Stimulationsfrequenz zwischen einer oberen und einer unteren Grenze ändert, *dadurch gekennzeichnet*, daß die Stimulationsfrequenz unterhalb der oberen Grenzfrequenz ist, wenn die Temperatur eine Grenztemperatur übersteigt.

2. Verfahren nach Anspruch 1, dadurch gekennzeichnet, daß die gemessene Temperatur diejenige des Blutes des Patienten ist.

3. Verfahren nach Anspruch 2, dadurch gekennzeichnet, daß die Temperatur im Herzen des Patienten gemessen wird.

4. Verfahren nach einem der Ansprüche 1 bis 3, dadurch gekennzeichnet, daß die Grenztemperatur zwischen 38° und 39°C, vorzugsweise gleich oder im Bereich von 38,5°C liegt.

5. Verfahren nach einem der Ansprüche 1 bis 4, dadurch gekennzeichnet, daß die untere Frequenz um 15 bis 25 %, vorzugsweise um 20 % niedriger als die obere Grenzfrequenz liegt.

6. Implantierbarer Herzschrittmacher, Mitteln (25, 17, 19) zum Messen der Temperatur eines Patienten, mit Mitteln (1, 2), die auf eine Änderung der Temperatur zum Erhöhen oder Verringern der Stimulationsfrequenz ansprechen, und mit Mitteln zum Vorsehen einer oberen und unteren Grenze für die Stimulationsfrequenz, dadurch gekennzeichnet, daß er mit Mitteln zum Zuweisen einer Frequenz unterhalb der oberen Grenzfrequenz dann, wenn die Temperatur eine Grenztemperatur übersteigt, versehen ist.

7. Herzschrittmacher nach Anspruch 6, dadurch gekennzeichnet, daß die Messmittel einen Thermistor (25) aufweisen, der mit einem elektronischen Schaltkreis (17, 19) verbunden ist und der zum Messen der Temperatur des Blutes des Patienten geeignet ist.

8. Herzschrittmacher nach Anspruch 7, dadurch gekennzeichnet, daß der Thermistor (25) von der Stimulationssonde (22), die mit dem Herzschrittmacher verbunden ist, gehalten ist.

9. Herzschrittmacher nach einem der Ansprüche 6 bis 8, dadurch gekennzeichnet, daß der elektronische Schaltkreis, der mit dem Thermistor (25) verbunden ist, einen Analog/Digital-Wandler (17) aufweist, der mit einem Zähler (19) verbunden ist, welcher mit einem Mikroprozessor (1) über einen Bus (20) verbunden ist, welcher Mikroprozessor (1) mit dem Zähler (19) über eine Steuerleitung (21) verbunden ist.

**Claims**

1. Method of controlling an implantable cardiac pacemaker, in which:

the heart is stimulated at a basic frequency corresponding to the patient's demand for a normal degree of physical activity;

the patient's temperature is measured as a parameter linked to the degree of physical activity of the patient, and

the stimulating frequency is modified in such a way that the stimulating frequency is increased or reduced when the temperature is respectively increased or reduced, the stimulating frequency varies between upper and lower limits,

characterized in that the stimulating frequency is below the upper limiting frequency if the temperature exceeds a limiting temperature.

2. Method according to Claim 1, characterized in that the temperature measured is that of the patient's blood.

3. Method according to Claim 2, characterized in that the temperature is measured in the patient's heart.

4. Method according to any one of Claims 1 to 3, characterized in that the limiting temperature is between 38 and 39°C, preferably equal to or in the neighbourhood of 38.5°C.

5. Method according to any one of Claims 1 to 4, characterized in that the low frequency is 15 to 25 %, preferably 20 % below the upper limiting frequency.

6. Implantable cardiac pacemaker comprising means (25, 17, 19) for measuring the patient's temperature, means (1, 2) responding to the change of temperature in order to increase or reduce the stimulating frequency, and means for setting upper and lower limits to the said stimulating frequency, characterized in that it has means for imposing a frequency below the upper limiting frequency if the temperature exceeds a limiting temperature.

7. Cardiac pacemaker according to Claim 6, characterized in that the measuring means comprise a thermistor (25) connected to an electronic circuit (17, 19) designed to measure the temperature of the patient's blood.

8. Cardiac pacemaker according to Claim 7, characterized in that the thermistor (25) is carried by the stimulating probe (32) connected to the cardiac pacemaker.

9. Cardiac pacemaker according to any one of Claims 6 to 8, characterized in that the electronic circuit connected to the thermistor (25) comprises an analog/digital converter (17) connected to a counter (19) which is connected to a microprocessor (1) by means of a bus (20), the microprocessor (1) being connected to the counter (19) by means of a control line (21).

FIG.1

FIG.2

FIG.3